# EUROPEAN PATENT SPECIFICATION

(11) **EP 4 351 426 B1**
(45) Date of publication and mention of the grant of the patent: **09.10.2024**
(21) Application number: 22748230.4
(22) Date of filing: 12.07.2022
(51) Int. Cl.: A61B 6/00

(54) **X-RAY IMAGE ACQUISITION METHOD AND SYSTEM, X-RAY MACHINE, AND STORAGE MEDIUM**
RÖNTGENBILDERFASSUNGSVERFAHREN UND -SYSTEM, RÖNTGENMASCHINE UND SPEICHERMEDIUM
PROCÉDÉ ET SYSTÈME D'ACQUISITION D'IMAGE RADIOGRAPHIQUE, MACHINE À RAYONS X ET SUPPORT DE STOCKAGE

(30) Priority: 13.07.2021 CN 202110788415
(43) Date of publication of application: 17.04.2024
(73) Proprietor: Siemens Shanghai Medical Equipment Ltd., Nanhui District Shanghai 201318 (CN)
(72) Inventor: WANG, Yudan, Shanghai 201318 (CN); FEI, Xiaoai, Shanghai 201612 (CN); YANG, Yousheng, Shanghai 201108 (CN); YANG, Zhiming, Shanghai 200030 (CN)
(74) Representative: Horn Kleimann Waitzhofer Schmid-Dreyer Patent- und Rechtsanwälte PartG mbB
(86) International application number: PCT/CN2022/105230
(87) International publication number: WO 2023/284746

(56) References cited:
- JP-A- 2000 100 597
- KR-B1- 101 025 165
- US-A1- 2016 192 892

## Description

### TECHNICAL FIELD

The present invention relates to the medical field, and in particular, to an X-ray image acquisition method and system, an X-ray machine, and a computer-readable storage medium.

### BACKGROUND

In a medical angiography X-ray machine, an X-ray source and an X-ray receiver (for example, a flat panel detector) are installed opposite to each other, so that X-rays generated by the X-ray source penetrate an object, and are then incident on and detected by the X-ray receiver.

However, in practical applications, local image overexposure caused by large density differences of human tissues in a target region of interest is a common image quality problem, that is, an image part of low-density tissues or a thinner body part is overexposed, while an image part of high-density tissues or a thicker body part is over-dark. In this case, it is difficult to see all details simultaneously. For example, during an interventional treatment procedure, lower-density regions are often overexposed, such as lung or air overexposure, which results in larger clinical image contrast. Especially in a cardiac interventional operation, lung regions are overexposed more obviously, which affects the visibility of a stent, and a precise position of the stent cannot be acquired in the overexposed regions. In another example, when legs are scanned, the region between the legs in the image is overexposed. In still another example, when a cervical spine is scanned, both sides of the cervical spine are prone to overexposure.

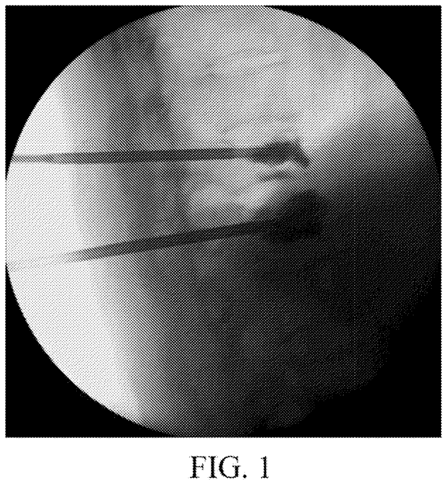

FIG. 1 shows an X-ray image of thoracic and lumbar spines of a patient. As shown in FIG. 1, the thoracic spine part of the image is overexposed, while the lumbar spine part of the image is over-dark, making it difficult for doctors to simultaneously see the thoracic and lumbar spines clearly. However, in fact, it often needs to acquire information about both the thoracic and lumbar spines.

In this case, to deal with different filtering requirements, at present, filters with different thicknesses are disposed between the X-ray source and the X-ray receiver to filter different amounts of X-rays, so that different filters are switched to achieve ideal image requirements.

In addition, those skilled in the art are still working to find other solutions.

US 2016/192892 A1 discloses a multiple frames imaging system with capability of differential x-ray exposure of different input areas of an image intensifier or other x-ray detector. Collimators are provided to control the amount of radiation in various regions of the image and image processing is provided to provide the display of images of different qualities. Motion methods are provided to move the collimators to produce optimal image frames.

JP 2000/100597 A discloses that when an X-ray controller indicates preparation for image pickup, a plane detector controller returns an exposure OK signal to the X-ray controller. During X-ray exposure, the plane detector controller periodically reads the pixels of a predetermined line of a plane detector and stores the result in an adder memory for each pixel. The pixel values are added, overwritten and stored in the memory. Each time when the whole pixels are written, a part of pixel value is read and inputted to a computing circuit. The sum value computed in the computing circuit is compared with a predetermined value, and it is reported to the X-ray controller when the sum value exceeds a threshold. The X-ray controller stops the X-ray irradiation from an X-ray tube. The plane detector controller successively outputs the signals of pixels stored in the plane detector.

KR101025165 B1 discloses a method for calculating an X-ray irradiation dose indicator for preventing the increase of an irradiation dose in case of photographing X-ray to prevent dose drift or exposure creep phenomenon by supplying objective recommendation tube current amount.

### SUMMARY

In view of this, one aspect of embodiments of the present invention provides an X-ray image acquisition method, and another aspect provides an X-ray image acquisition system, an X-ray machine, and a computer-readable storage medium, which are used to acquire images with high imaging quality for human tissues with large density differences.

The X-ray image acquisition method provided in the embodiments of the present invention includes: acquiring a captured target image of a target region; when it is determined that there is at least one to-be-optimized region in the target image, calculating, for each to-be-optimized region, a pixel average of the to-be-optimized region in the target image; determining a to-be-optimized region received dose corresponding to the pixel average of the to-be-optimized region according to a relationship between a predetermined pixel average and an X-ray received dose of an X-ray receiver; adjusting an X-ray emission dose of an X-ray source according to a principle of making the to-be-optimized region received dose reach a predetermined X-ray reference received dose, and acquiring an optimized image of the target region captured based on the X-ray emission dose adjusted to meet a requirement, where the at least one to-be-optimized region includes an overexposed bright region and/or an underexposed dark region; and adding and synthesizing at least one optimized image corresponding to the at least one to-be-optimized region, or adding and synthesizing at least one optimized image corresponding to the at least one to-be-optimized region and the target image, to obtain an X-ray image of the target region.

In an implementation, the at least one to-be-optimized region includes an overexposed bright region and an underexposed dark region; and the calculating, for each to-be-optimized region, a pixel average of the to-be-optimized region in the target image includes: calculating a histogram of the target image; finding a global threshold of the histogram by using a threshold segmentation method; and calculating, based on the global threshold, an average of pixels in the histogram with values less than the global threshold, to obtain a dark region pixel average; and calculating an average of pixels in the histogram with values greater than the global threshold, to obtain a bright region pixel average.

In an implementation, the adding and synthesizing at least one optimized image corresponding to the at least one to-be-optimized region, or adding and synthesizing at least one optimized image corresponding to the at least one to-be-optimized region and the target image includes: entirely superimposing the at least one optimized image corresponding to the at least one to-be-optimized region, or synthesizing image parts corresponding to respective to-be-optimized regions in optimized images corresponding to different to-be-optimized regions.

In an implementation, the at least one to-be-optimized region includes an overexposed bright region and/or an underexposed dark region; and the calculating, for each to-be-optimized region, a pixel average of the to-be-optimized region in the target image includes: calculating an average of pixels in the target image with brightness values greater than a first brightness threshold, to obtain a bright region pixel average; and/or calculating an average of pixels in the target image with brightness values less than a second brightness threshold, to obtain a dark region pixel average, where the second brightness threshold is less than or equal to the first brightness threshold.

In an implementation, the at least one to-be-optimized region includes an overexposed bright region and an underexposed dark region, and the second brightness threshold is equal to the first brightness threshold; and the adding and synthesizing at least one optimized image corresponding to the at least one to-be-optimized region, or adding and synthesizing at least one optimized image corresponding to the at least one to-be-optimized region and the target image includes: entirely superimposing the at least one optimized image corresponding to the at least one to-be-optimized region, or synthesizing image parts corresponding to respective to-be-optimized regions in optimized images corresponding to different to-be-optimized regions; or the at least one to-be-optimized region includes an overexposed bright region and an underexposed dark region, and the second brightness threshold is less than the first brightness threshold; or the at least one to-be-optimized region includes an overexposed bright region or an underexposed dark region, and either the bright region or the dark region is part of the target image; and the adding and synthesizing at least one optimized image corresponding to the at least one to-be-optimized region, or adding and synthesizing at least one optimized image corresponding to the at least one to-be-optimized region and the target image includes: entirely superimposing the at least one optimized image corresponding to the at least one to-be-optimized region and the target image, or synthesizing image parts corresponding to respective to-be-optimized regions in optimized images corresponding to different to-be-optimized regions and image parts in the target image other than the at least one to-be-optimized region.

In an implementation, the method further includes: detecting the pixels in the target image with the brightness values greater than the preset first brightness threshold to obtain overexposed pixels, and determining that there is an overexposed bright region in the target image when a quantity of the overexposed pixels reaches a preset first quantity threshold; and/or detecting the pixels in the target image with the brightness values less than the preset second brightness threshold to obtain over-dark pixels, and determining that there is a to-be-optimized underexposed dark region in the target image when a quantity of the over-dark pixels reaches a preset second quantity threshold, where the second brightness threshold is less than or equal to the first brightness threshold.

The X-ray image acquisition system provided in the embodiments of the present invention includes: a first unit, configured to acquire a captured target image of a target region; a second unit, configured to, when it is determined that there is at least one to-be-optimized region in the target image, calculate, for each to-be-optimized region, a pixel average of the to-be-optimized region in the target image; determine a to-be-optimized region received dose corresponding to the pixel average of the to-be-optimized region according to a relationship between a predetermined pixel average and an X-ray received dose of an X-ray receiver; and adjust an X-ray emission dose of an X-ray source according to a principle of making the to-be-optimized region received dose reach a predetermined X-ray reference received dose, and acquire an optimized image of the target region captured based on the X-ray emission dose adjusted to meet a requirement, where the at least one to-be-optimized region includes an overexposed bright region and/or an underexposed dark region; and a third unit, configured to add and synthesize at least one optimized image corresponding to the at least one to-be-optimized region, or add and synthesize at least one optimized image corresponding to the at least one to-be-optimized region and the target image, to obtain an X-ray image of the target region.

In an implementation, the at least one to-be-optimized region includes an overexposed bright region and an underexposed dark region; and the second unit is configured to calculate a histogram of the target image; find a global threshold of the histogram by using a threshold segmentation method; and calculate, based on the global threshold, an average of pixels in the histogram with values less than the global threshold, to obtain a dark region pixel average; and calculate an average of pixels in the histogram with values greater than the global threshold, to obtain a bright region pixel average.

In an implementation, the third unit is configured to entirely superimpose the at least one optimized image corresponding to the at least one to-be-optimized region, or synthesize image parts corresponding to respective to-be-optimized regions in optimized images corresponding to different to-be-optimized regions, to obtain the X-ray image of the target region.

In an implementation, the at least one to-be-optimized region includes an overexposed bright region and/or an underexposed dark region; and the second unit is configured to calculate an average of pixels in the target image with brightness values greater than a first brightness threshold, to obtain a bright region pixel average; and/or calculate an average of pixels in the target image with brightness values less than a second brightness threshold, to obtain a dark region pixel average.

In an implementation, the at least one to-be-optimized region includes the overexposed bright region and the underexposed dark region, and the second brightness threshold is equal to the first brightness threshold; and the third unit is configured to entirely superimpose the at least one optimized image corresponding to the at least one to-be-optimized region, or synthesize image parts corresponding to respective to-be-optimized regions in optimized images corresponding to different to-be-optimized regions, to obtain the X-ray image of the target region; or the at least one to-be-optimized region includes an overexposed bright region and an underexposed dark region, and the second brightness threshold is less than the first brightness threshold; or the at least one to-be-optimized region includes an overexposed bright region or an underexposed dark region, and either the bright region or the dark region is part of the target image; and the third unit is configured to entirely superimpose the at least one optimized image corresponding to the at least one to-be-optimized region and the target image, or synthesize image parts corresponding to respective to-be-optimized regionsin optimized images corresponding to different to-be-optimized regions and image parts in the target image other than the at least one to-be-optimized region.

In an implementation, the method further includes: a fourth unit, configured to detect the pixels in the target image with the brightness values greater than the preset first brightness threshold to obtain overexposed pixels, and determine that there is an overexposed bright region in the target image when a quantity of the overexposed pixels reaches a preset first quantity threshold; and/or detect the pixels in the target image with the brightness values less than the preset second brightness threshold to obtain over-dark pixels, and determine that there is an underexposed dark region in the target image when a quantity of the over-dark pixels reaches a preset second quantity threshold, where the second brightness threshold is less than or equal to the first brightness threshold.

Another X-ray image acquisition system provided in the embodiments of the present invention includes: at least one memory and at least one processor, where the at least one memory is configured to store a computer program; and the at least one processor is configured to call the computer program stored in the at least one memory to perform the X-ray image acquisition method according to any one of the foregoing implementations.

The X-ray machine provided in the embodiments of the present invention includes: the X-ray image acquisition system according to any one of the foregoing implementations.

The computer-readable storage medium provided in the embodiments of the present invention stores a computer program, where the computer program is executable by a processor to implement the X-ray image acquisition method according to any one of the foregoing implementations.

As can be seen from the foregoing solutions, in the embodiments of the present invention, when it is determined that there are to-be-optimized regions including an overexposed bright region and/or an underexposed dark region in the target image, for each to-be-optimized region, the X-ray emission dose is adjusted according to the X-ray received dose that meets the imaging quality requirement, the optimized images are acquired based on the X-ray emission dose adjusted to meet the requirement, and the optimized images are then added and synthesized to obtain the X-ray image that meets the imaging quality requirement as a whole.

In addition, whether there is a to-be-optimized region in the target image is automatically detected by the system, which can improve the intelligence and flexibility of the system application.

### BRIEF DESCRIPTION OF THE DRAWINGS

To enable a person of ordinary skill in the art to understand the foregoing and other features and advantages of the present invention more clearly, exemplary embodiments of the present invention are described in detail below with reference to the accompanying drawings. In the accompanying drawings:
FIG. 1 shows an X-ray image of thoracic and lumbar spines of a patient.
FIG. 2 is an exemplary flowchart of an X-ray image acquisition method according to an embodiment of the present invention.
FIG. 3 is a schematic diagram of a to-be-scanned body model in an example of the present invention.
FIG. 4A is a schematic diagram of a target image in an example of the present invention.
FIG. 4B is a schematic diagram of a histogram of the target image shown in FIG. 4A and a global threshold TS found based on the histogram.
FIG. 4C and FIG. 4D are respectively schematic diagrams of a first optimized image and a second optimized image obtained by performing local image optimization based on the body model shown in FIG. 3.
FIG. 4E is a schematic diagram of an X-ray image obtained by adding the first optimized image shown in FIG. 4C and the second optimized image shown in FIG. 4D.
FIG. 5 is an exemplary structural diagram of an X-ray image acquisition system according to an embodiment of the present invention.
FIG. 6 is an exemplary structural diagram of another X-ray image acquisition system according to an embodiment of the present invention.

Reference numerals are as follows:

| Number | Meaning |
|---|---|
| S21 to S25 | Steps |
| 31 | Pelvis model |
| 32 | Elbow model |
| 510 | First unit |
| 520 | Second unit |
| 530 | Third unit |
| 540 | Fourth unit |
| 61 | Memory |
| 62 | Processor |
| 63 | Display |
| 64 | Bus |

### DETAILED DESCRIPTION

In consideration of a limited dynamic range of an X-ray receiver, it is difficult to record all signals using a high signal-to-noise ratio. For a high absorption part of X-rays in human tissues, a dark region with high noise, that is, an underexposed region, may appear in an image; while for a low absorption part, a bright region, that is, an overexposed region, may appear in the image. Therefore, in the embodiments of the present invention, it is considered to obtain a final image by combining a plurality of images, and these images may be images optimized for different image parts. For example, when there are both a bright region and a dark region in images, one image may be focused on the dark region and the other on the bright region. The images are then added and synthesized to obtain a final image with good image quality, and the image can clearly present information about the dark region and the bright region.

For clearer understanding of the objectives, technical solutions, and effects of the present invention, specific implementations of the present invention are now illustrated with reference to the accompanying drawings, and same reference numerals in the accompanying drawings represent components with same structures or with similar structures and same functions.

In this specification, "exemplary" and "schematic" indicate "serving as an example, a case, or description", and any illustration or implementation described as "exemplary" and "schematic" in this specification should not be interpreted as a more preferred or more advantageous technical solution.

For brevity of the accompanying drawings, only parts related to the present invention are schematically shown in the accompanying drawings, and do not represent an actual structure as a product.

In this specification, "one" not only indicates "only one", but also indicates "more than one". In this specification, "first", "second", and the like are only used to distinguish from each other, but not to indicate importance, an order, and the like.

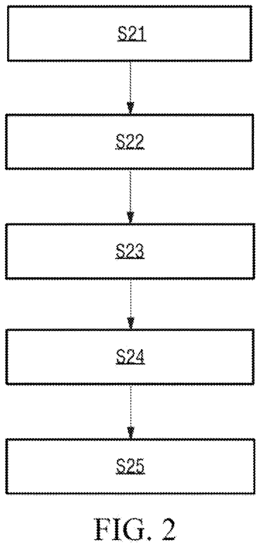

FIG. 2 is an exemplary flowchart of an X-ray image acquisition method according to an embodiment of the present invention. As shown in FIG. 2, the method may include the following steps:
Step S21: Acquire a captured target image of a target region.

In this step, the target region may be a region of interest of a user in a clinical operation. In addition, the target region may be changed according to actual applications, and when the target region is changed, the image acquired in step S21 is also changed correspondingly.

During specific implementation, the solution in this embodiment may be performed in real time, and correspondingly, step S21 is also performed in real time; or the solution may be performed periodically, and correspondingly, step S21 is also performed periodically; or the solution may be performed by triggering a condition, and correspondingly, step S21 may be triggered when the target region is initially determined and when the target region is changed.

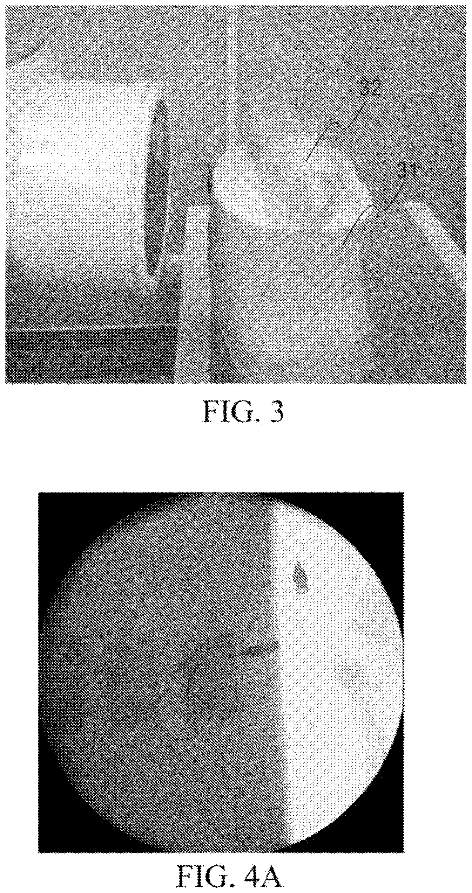

As shown in FIG. 3, a pelvis model 31 and an elbow model 32 are put together to simulate bodies with different thicknesses, thereby obtaining a schematic diagram of a target image shown in FIG. 4A. The pelvis is a high absorption part of X-rays, and the elbow is a low absorption part of X-rays.

Step S22: When it is determined that there is at least one to-be-optimized region such as an overexposed bright region and/or an underexposed dark region in the target image, calculate a pixel average such as a bright region pixel average and/or a dark region pixel average of the to-be-optimized region in the target image.

In this step, there may be various methods for determining whether there is an overexposed bright region and/or an underexposed dark region in the target image. For example, if the user can directly determine based on experience that there may be a to-be-optimized bright region and/or dark region in the image of the target region, the system can directly determine a to-be-optimized region, for example, according to an instruction or settings of the user. Correspondingly, in this embodiment, a pixel average such as a bright region pixel average and/or dark region pixel average of a to-be-optimized region in the target image can be directly calculated. For an application scenario in which the target region is constantly changed, or for the convenience of users with insufficient experience, or to improve the application flexibility of the method, a to-be-optimized region can be alternatively automatically determined by the system. That is, in this embodiment, before a pixel average such as a bright region pixel average and/or dark region pixel average of a to-be-optimized region in the target image is calculated, the method may further include: detecting whether there is a to-be-optimized region such as an overexposed bright region and/or an underexposed dark region in the target image. For example, during specific implementation, pixels in the target image with brightness values greater than a preset first brightness threshold can be detected based on a histogram technology, to obtain overexposed pixels, and it is determined that there is a to-be-optimized overexposed bright region in the target image when a quantity of the overexposed pixels reaches a preset first quantity threshold; otherwise it can be determined that there is no to-be-optimized overexposed bright region in the target image; and/or, pixels in the target image with brightness values less than a preset second brightness threshold are detected based on the histogram technology, to obtain over-dark pixels, and it is determined that there is a to-be-optimized underexposed dark region in the target image when a quantity of the over-dark pixels reaches a preset second quantity threshold; otherwise it can be determined that there is no to-be-optimized underexposed dark region in the target image. The second brightness threshold is less than or equal to the first brightness threshold. In addition, in other implementations, an image gradient calculation algorithm can be further used to determine a grayscale gradient distribution of a bright region and/or a dark region, an edge of the bright region and/or the dark region is further detected, and edge curves are then used to fit a bright region boundary and/or a dark region boundary.

There may be various methods for calculating a pixel average such as a bright region pixel average and/or a dark region pixel average of a to-be-optimized region in the target image.

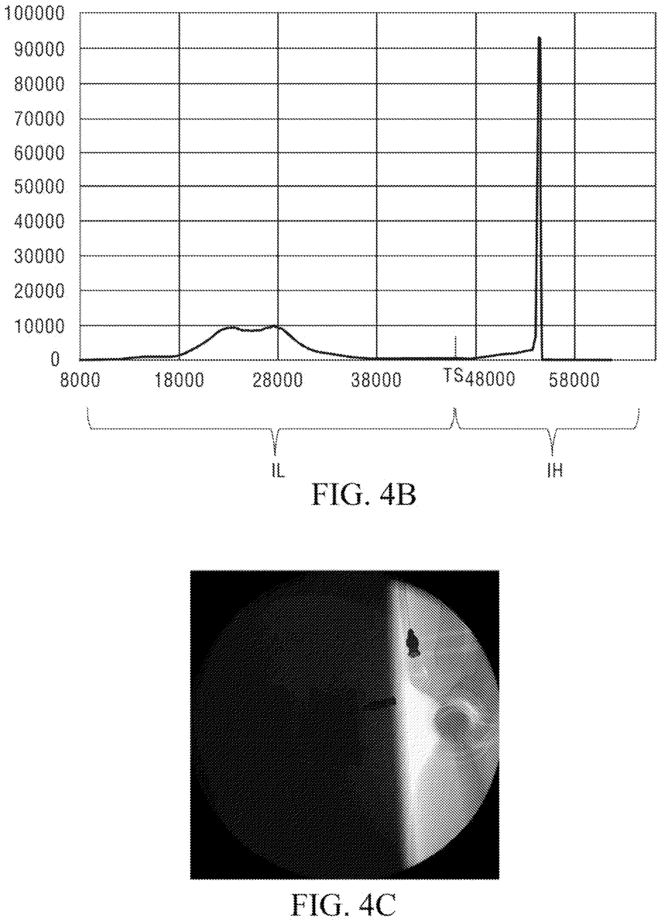

For example, if it is determined that there are both an overexposed bright region and an underexposed dark region to be optimized in the target image, in a first example, a histogram of the target image can be calculated first, and a global threshold of the histogram can be found by using a threshold segmentation method such as a maximum between-class variance method (also referred to as the OSTU method). FIG. 4B is a schematic diagram of a histogram of the target image shown in FIG. 4A and a global threshold TS found based on the histogram. An average of pixels IH in the histogram with values greater than the global threshold can be then calculated based on the global threshold, to obtain a bright region pixel average; and an average of pixels IL in the histogram with values less than the global threshold can be calculated, to obtain a dark region pixel average.

In a second example, an average of pixels in the target image with brightness values greater than a first brightness threshold can be calculated based on the histogram technology, to obtain a bright region pixel average; and an average of pixels in the target image with brightness values less than a second brightness threshold can be calculated, to obtain a dark region pixel average. The second brightness threshold is less than or equal to the first brightness threshold.

If it is determined that there is a to-be-optimized overexposed bright region in the target image, but there is no underexposed dark region, or even if there is a dark region, but the optimization on the dark region is not considered, an average of pixels in the target image with brightness values greater than a first brightness threshold can be calculated based on the histogram technology, to obtain a bright region pixel average.

If it is determined that there is a to-be-optimized underexposed dark region in the target image, but there is no overexposed bright region, or even if there is a bright region, but the optimization on the bright region is not considered, an average of pixels in the target image with brightness values less than a second brightness threshold can be calculated based on the histogram technology, to obtain a dark region pixel average.

Certainly, when it is determined that there is no overexposed bright region or underexposed dark region to be optimized in the target image, the target image can be directly used as an X-ray image of the target region.

Step S23: Determine a to-be-optimized region received dose corresponding to the pixel average of the to-be-optimized region according to a relationship between a predetermined pixel average and an X-ray received dose of an X-ray receiver, for example, determine a bright region received dose corresponding to the bright region pixel average and/or a dark region received dose corresponding to the dark region pixel average.

In this step, if there are both an overexposed bright region and an underexposed dark region to be optimized in the target image, a bright region received dose corresponding to the bright region pixel average and a dark region received dose corresponding to the dark region pixel average are determined according to a relationship between a predetermined pixel average and an X-ray received dose of an X-ray receiver.

If there is a to-be-optimized overexposed bright region in the target image, but there is no underexposed dark region, or even if there is a dark region, but the optimization on the dark region is not considered, a bright region received dose corresponding to the bright region pixel average is determined according to a relationship between a predetermined pixel average and an X-ray received dose of an X-ray receiver.

If there is a to-be-optimized underexposed dark region in the target image, but there is no overexposed bright region, or even if there is a bright region, but the optimization on the bright region is not considered, a dark region received dose corresponding to the dark region pixel average is determined according to a relationship between a predetermined pixel average and an X-ray received dose of an X-ray receiver.

Step S24: Compare the to-be-optimized region received dose with a predetermined X-ray reference received dose that meets an imaging requirement, adjust an X-ray emission dose of an X-ray source according to a principle of making the to-be-optimized region received dose reach the X-ray reference received dose, and acquire an optimized image of the target region captured based on the X-ray emission dose adjusted to meet the requirement. For example, the bright region received dose is compared with a predetermined X-ray reference received dose that meets an imaging requirement, an X-ray emission dose of an X-ray source is adjusted according to a principle of making the bright region received dose reach the X-ray reference received dose or close to the X-ray reference received dose by a preset degree, and a first optimized image of the target region captured based on the X-ray emission dose adjusted to meet the requirement is acquired; and/or, the dark region received dose is compared with the X-ray reference received dose, an X-ray emission dose of an X-ray source is adjusted according to a principle of making the dark region received dose reach the X-ray reference received dose or close to the X-ray reference received dose by a preset degree, and a second optimized image of the target region captured based on the X-ray emission dose adjusted to meet the requirement is acquired.

In this embodiment, the X-ray reference received dose may be a value range, such as A ± 10% or A ± 5%, where A is the predetermined X-ray received dose that meets the imaging requirement, for example, 100.

The process of adjusting an X-ray emission dose of an X-ray source according to a principle of making the to-be-optimized region received dose reach the X-ray reference received dose may be a cyclic process, which is because one adjustment may not guarantee that the corresponding X-ray received dose can reach the X-ray reference received dose. Therefore, after one adjustment is performed, a current adjusted image of the target region captured based on the adjusted X-ray emission dose can be acquired, and a pixel average of a to-be-optimized region in the current adjusted image can be calculated. A to-be-optimized region received dose corresponding to the pixel average of the to-be-optimized region is determined according to a relationship between the pixel average and an X-ray received dose of an X-ray receiver. Whether the to-be-optimized region received dose reaches the X-ray reference received dose is determined, and if yes, the current adjusted image is used as an optimized image corresponding to the to-be-optimized region; otherwise the X-ray emission dose of the X-ray source is adjusted again, and the process returns to the step of acquiring a current adjusted image of the target region captured based on the adjusted X-ray emission dose. The adjustment of the X-ray emission dose of the X-ray source can be implemented by adjusting parameters such as voltage and current of the X-ray source.

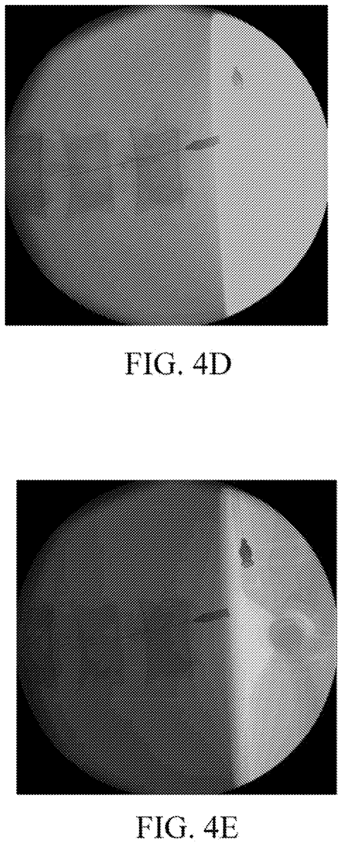

In this step, if there are both an overexposed bright region and an underexposed dark region to be optimized in the target image, a first optimized image and a second optimized image are acquired. FIG. 4C and FIG. 4D are respectively schematic diagrams of a first optimized image and a second optimized image obtained by performing local image optimization based on the body model shown in FIG. 3.

If there is a to-be-optimized overexposed bright region in the target image, but there is no underexposed dark region, or even if there is a dark region, but the optimization on the dark region is not considered, only the first optimized image is acquired.

If there is a to-be-optimized underexposed dark region in the target image, but there is no overexposed bright region, or even if there is a bright region, but the optimization of the bright region is not considered, only the second optimized image is acquired.

Step S25: Add and synthesize at least one optimized image corresponding to the at least one to-be-optimized region, or add and synthesize at least one optimized image corresponding to the at least one to-be-optimized region and the target image, to obtain an X-ray image of the target region. For example, the first optimized image and the second optimized image are added and synthesized, or the first optimized image and/or the second optimized image and the target image are added and synthesized, to obtain an X-ray image of the target region.

In this step, if there are both an overexposed bright region and an underexposed dark region to be optimized in the target image, and a bright region pixel average and a dark region pixel average are acquired by using the method in the first example in step S22, the first optimized image and the second optimized image can be directly added and synthesized to obtain an X-ray image of the target region. Specifically, the at least one optimized image corresponding to the at least one to-be-optimized region can be entirely superimposed. FIG. 4E shows an X-ray image obtained by entirely superimposing the first optimized image shown in FIG. 4C and the second optimized image shown in FIG. 4D. It can be seen that the optimized and recombined image is a clear X-ray image that can present tissues with large density differences. Certainly, in other implementations, image parts corresponding to respective to-be-optimized regions in optimized images corresponding to different to-be-optimized regions can be alternatively synthesized. For example, an image part corresponding to a to-be-optimized region in an optimized image can be segmented based on the bright region boundary and/or the dark region boundary, and the segmented parts are synthesized to obtain an X-ray image of the target region.

If there are both an overexposed bright region and an underexposed dark region to be optimized in the target image, and a bright region pixel average and a dark region pixel average are acquired by using the method in the second example in step S22, but the second brightness threshold is equal to the first brightness threshold, the first optimized image and the second optimized image can still be directly added and synthesized as described above to obtain an X-ray image of the target region. If a bright region pixel average and a dark region pixel average are acquired by using the method in the second example in step S22, but the second brightness threshold is less than the first brightness threshold, the first optimized image, the second optimized image, and the target image can be added and synthesized to obtain an X-ray image of the target region.

If there is a to-be-optimized overexposed bright region in the target image, but there is no underexposed dark region, or even if there is a dark region, but the optimization on the dark region is not considered, the first optimized image and the target image can be added and synthesized to obtain an X-ray image of the target region.

If there is a to-be-optimized underexposed dark region in the target image, but there is no overexposed bright region, or even if there is a bright region, but the optimization on the bright region is not considered, the second optimized image and the target image can be added and synthesized to obtain an X-ray image of the target region.

The adding and synthesizing at least one optimized image corresponding to the at least one to-be-optimized region and the target image may be entirely superimposing the at least one optimized image corresponding to the at least one to-be-optimized region and the target image, or synthesizing image parts corresponding to respective to-be-optimized regions in optimized images corresponding to different to-be-optimized regions and image parts in the target image other than the at least one to-be-optimized region. For example, an image part corresponding to a to-be-optimized region in an optimized image and an image part in the target image other than the at least one to-be-optimized region can be segmented based on the bright region boundary and/or the dark region boundary, and the segmented parts are then synthesized to obtain an X-ray image of the target region.

The foregoing steps S21 to S25 in this embodiment are not used to limit the execution order of the technical features. For example, if there are both an overexposed bright region and an underexposed dark region to be optimized in the target image, in addition to the execution order shown in step S21 to step S25 in FIG. 2, the execution order in this embodiment may be alternatively: calculating a bright region pixel average in the target image; determining a bright region received dose corresponding to the bright region pixel average according to a relationship between a predetermined pixel average and an X-ray received dose of an X-ray receiver; and comparing the bright region received dose with a predetermined X-ray reference received dose that meets an imaging requirement, adjusting an X-ray emission dose of an X-ray source according to a principle of making the bright region received dose reach the X-ray reference received dose or close to the X-ray reference received dose by a preset degree, and acquiring a first optimized image of the target region captured based on the adjusted X-ray emission dose; calculating a dark region pixel average in the target image; determining a dark region received dose corresponding to the dark region pixel average according to a relationship between a predetermined pixel average and an X-ray received dose of an X-ray receiver; and comparing the dark region received dose with an X-ray reference received dose, adjusting an X-ray emission dose of an X-ray source according to a principle of making the dark region received dose reach the X-ray reference received dose or close to the X-ray reference received dose by a preset degree, and acquiring a second optimized image of the target region captured based on the adjusted X-ray emission dose. In addition, the execution order may be alternatively: calculating a dark region pixel average in the target image; determining a dark region received dose corresponding to the dark region pixel average according to a relationship between a predetermined pixel average and an X-ray received dose of an X-ray receiver; and comparing the dark region received dose with an X-ray reference received dose, adjusting an X-ray emission dose of an X-ray source according to a principle of making the dark region received dose reach the X-ray reference received dose or close to the X-ray reference received dose by a preset degree, and acquiring a second optimized image of the target region captured based on the adjusted X-ray emission dose; calculating a bright region pixel average in the target image; determining a bright region received dose corresponding to the bright region pixel average according to a relationship between a predetermined pixel average and an X-ray received dose of an X-ray receiver; and comparing the bright region received dose with the predetermined X-ray reference received dose that meets an imaging requirement, adjusting an X-ray emission dose of an X-ray source according to a principle of making the bright region received dose reach the X-ray reference received dose or close to the X-ray reference received dose by a preset degree, and acquiring a first optimized image of the target region captured based on the adjusted X-ray emission dose. The specific execution order can be adjusted according to actual requirements, and is not limited herein.

The X-ray image acquisition method in the embodiments of the present invention is described in detail above, and an X-ray image acquisition system in the embodiments of the present invention is described in detail below. The X-ray image acquisition system in the embodiments of the present invention may be used to implement the X-ray image acquisition method in the embodiments of the present invention. For details not disclosed in detail in the system embodiments of the present invention, refer to the corresponding descriptions in the method embodiments of the present invention, and the details are not repeated herein.

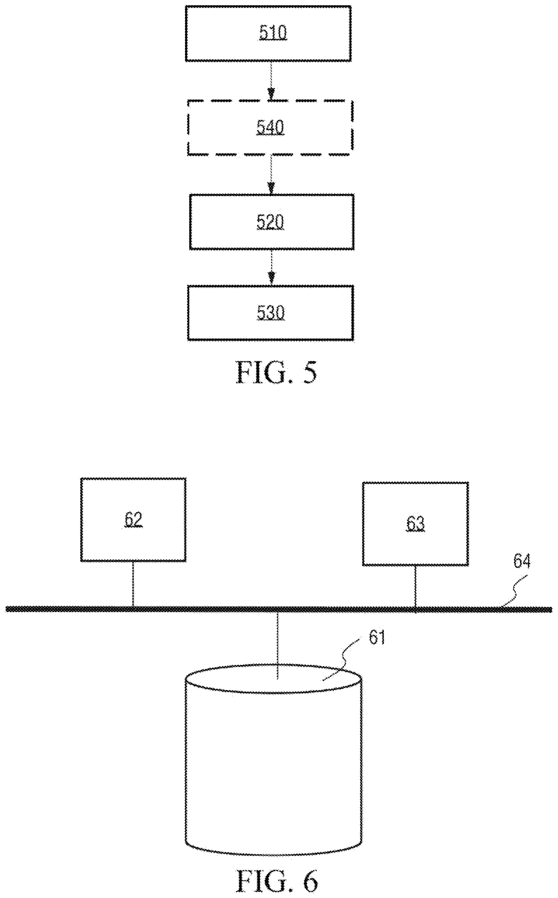

FIG. 5 is an exemplary structural diagram of an X-ray image acquisition system according to an embodiment of the present invention. As shown in the solid-line parts in FIG. 5, the system may include: a first unit 510, a second unit 520, and a third unit 530.

The first unit 510 is configured to acquire a captured target image of a target region.

The second unit 520 is configured to, when it is determined that there is at least one to-be-optimized region in the target image, calculate, for each to-be-optimized region, a pixel average of the to-be-optimized region in the target image; determine a to-be-optimized region received dose corresponding to the pixel average of the to-be-optimized region according to a relationship between a predetermined pixel average and an X-ray received dose of an X-ray receiver; and adjust an X-ray emission dose of an X-ray source according to a principle of making the to-be-optimized region received dose reach a predetermined X-ray reference received dose, and acquire an optimized image of the target region captured based on the X-ray emission dose adjusted to meet a requirement, where the at least one to-be-optimized region includes an overexposed bright region and/or an underexposed dark region. For example, when it is determined that there is a to-be-optimized overexposed bright region in the target image, a bright region pixel average in the target image is calculated; a bright region received dose corresponding to the bright region pixel average is determined according to a relationship between a predetermined pixel average and an X-ray received dose of an X-ray receiver; and the bright region received dose is compared with a predetermined X-ray reference received dose that meets an imaging requirement, an X-ray emission dose of an X-ray source is adjusted according to a principle of making the bright region received dose reach the X-ray reference received dose, and a first optimized image of the target region captured based on the X-ray emission dose adjusted to meet the requirement is acquired; and/or, when it is determined that there is a to-be-optimized underexposed dark region in the target image, a dark region pixel average in the target image is calculated; a dark region received dose corresponding to the dark region pixel average is determined according to a relationship between a predetermined pixel average and an X-ray received dose of an X-ray receiver; and the dark region received dose is compared with the X-ray reference received dose, an X-ray emission dose of an X-ray source is adjusted according to a principle of making the dark region received dose reach the X-ray reference received dose, and a second optimized image of the target region captured based on the X-ray emission dose adjusted to meet the requirement is acquired.

Corresponding to the method shown in FIG. 2, when it is determined that there are an overexposed bright region and an underexposed dark region to be optimized in the target image, the second unit 520 may perform the method in the first example in step S22: calculating a histogram of the target image; finding a global threshold of the histogram by using a threshold segmentation method such as the OSTU method; and calculating, based on the global threshold, an average of pixels in the histogram with values less than the global threshold, to obtain a dark region pixel average; and calculating an average of pixels in the histogram with values greater than the global threshold, to obtain a bright region pixel average. Alternatively, when it is determined that there are an overexposed bright region and/or an underexposed dark region to be optimized in the target image, the second unit 520 may perform the method in the second example in step S22: calculating an average of pixels in the target image with brightness values greater than a first brightness threshold based on a histogram technology, to obtain a bright region pixel average; and/or calculating an average of pixels in the target image with brightness values less than a second brightness threshold based on the histogram technology, to obtain a dark region pixel average.

The third unit 530 is configured to add and synthesize at least one optimized image corresponding to the at least one to-be-optimized region, or add and synthesize at least one optimized image corresponding to the at least one to-be-optimized region and the target image, to obtain an X-ray image of the target region. For example, the first optimized image and the second optimized image are added, or the first optimized image and/or the second optimized image and the target image are added, to obtain an X-ray image of the target region.

Corresponding to the method shown in FIG. 2, when it is determined that there are an overexposed bright region and an underexposed dark region to be optimized in the target image, and the second unit 520 performs the method in the first example in step S22 to calculate a pixel average of the to-be-optimized region and further obtain an optimized image, or when the second unit 520 performs the method in the second example in step S22 to calculate a pixel average of the to-be-optimized region and further obtain an optimized image, and the second brightness threshold is equal to the first brightness threshold, the third unit 530 may entirely superimpose the at least one optimized image corresponding to the at least one to-be-optimized region, or synthesize image parts corresponding to respective to-be-optimized regions in optimized images corresponding to different to-be-optimized regions, to obtain an X-ray image of the target region. When it is determined that there are an overexposed bright region and an underexposed dark region to be optimized in the target image, and the second unit 520 performs the method in the second example in step S22 to calculate a pixel average of the to-be-optimized region and further obtain an optimized image, if the second brightness threshold is less than the first brightness threshold, or when it is determined that there is only an overexposed bright region or an underexposed dark region to be optimized in the target image, the third unit 530 may entirely superimpose the at least one optimized image corresponding to the at least one to-be-optimized region and the target image, or synthesize image parts corresponding to respective to-be-optimized regionsin optimized images corresponding to different to-be-optimized regions and image parts in the target image other than the at least one to-be-optimized region, to obtain an X-ray image of the target region.

Corresponding to the method shown in FIG. 2, the system in the embodiments of the present invention may be shown in the dotted-line part in FIG. 5, and further includes: a fourth unit 540, configured to detect, based on the histogram technology, the pixels in the target image with the brightness values greater than the preset first brightness threshold to obtain overexposed pixels, and determine that there is a to-be-optimized overexposed bright region in the target image when a quantity of the overexposed pixels reaches a preset first quantity threshold; and/or detect, based on the histogram technology, the pixels in the target image with the brightness values less than the preset second brightness threshold to obtain over-dark pixels, and determine that there is a to-be-optimized underexposed dark region in the target image when a quantity of the over-dark pixels reaches a preset second quantity threshold, where the second brightness threshold is less than or equal to the first brightness threshold.

FIG. 6 is a schematic structural diagram of another X-ray image acquisition system according to an embodiment of the present invention. As shown in FIG. 6, the system may include: at least one memory 61, at least one processor 62, and at least one display 63. In addition, some other components such as a communication port may be further included. These components communicate by using a bus 64.

The at least one memory 61 is configured to store a computer program. In an implementation, the computer program may be understood as including various modules of the X-ray image acquisition system shown in FIG. 5. In addition, the at least one memory 61 may further store an operating system and the like. The operating system includes but is not limited to: an Android operating system, a Symbian operating system, a Windows operating system, a Linux operating system, and the like.

The at least one display 63 is configured to display a captured target image, a first optimized image and/or a second optimized image, a final X-ray image, and the like.

The at least one processor 62 is configured to call the computer program stored in the at least one memory 61 to perform the X-ray image acquisition method according to the embodiments of the present invention. The processor 62 may be a CPU, a processing unit/module, an ASIC, a logical module, a programmable gate array, or the like. The processor may receive and transmit data through the communication port.

An embodiment of the present invention further provides an X-ray machine, which includes the X-ray image acquisition system according to any one of the foregoing implementations.

It should be noted that, not all steps and modules in the procedures and the structural diagrams are necessary, and some steps or modules may be omitted according to an actual requirement. An execution order of the steps is not fixed and may be adjusted according to requirements. Division of the modules is merely functional division for ease of description. During actual implementation, one module may be implemented separately by a plurality of modules, and functions of the plurality of modules may alternatively be implemented by the same module. The modules may be located in the same device or in different devices.

It can be understood that hardware modules in the implementations may be implemented in a mechanic manner or an electronic manner. For example, a hardware module may include specially designed permanent circuits or logic devices (for example, an application-specific processor such as an FPGA or an ASIC) to complete specific operations. The hardware module may also include temporarily configured programmable logic devices or circuits (for example, including a universal processor or another programmable processor) to perform specific operations. The hardware module is implemented by specifically using the mechanical manner, using the application-specific permanent circuits, or using the temporarily configured circuits (for example, configured by software), which can be decided according to consideration of costs and time.

In addition, an embodiment of the present invention further provides a computer-readable storage medium, storing a computer program, where the computer program is executable by a processor to implement the X-ray image acquisition method according to the embodiments of the present invention. Specifically, a system or an apparatus that is equipped with a storage medium may be provided. The storage medium stores software program code that implements functions of any implementation in the foregoing embodiments, and a computer (a CPU or an MPU) of the system or the apparatus is enabled to read and execute the program code stored in the storage medium. In addition, a program code based instruction may also be used to enable an operating system or the like running in the computer to complete some or all actual operations. The program code read from the storage medium may also be written into a memory that is disposed in an expansion board inserted in the computer, or may be written into a memory that is disposed in an expansion unit connected to the computer, and then a CPU or the like that is installed on the expansion board or expansion unit may be enabled to execute some or all actual operations based on the instructions of the program code, so as to implement the functions of any one of the foregoing implementations. Implementations of the storage medium for providing the program code may include a floppy disk, a hard disk, a magneto-optical disk, an optical memory (such as a CD-ROM, a CD-R, a CD-RW, a DVD-ROM, a DVD-RAM, a DVD-RW, and a DVD+RW), a magnetic tape, a non-volatile storage card, and a ROM. Optionally, the program code may be downloaded from a server computer by using a communication network.

As can be seen from the foregoing solutions, in the embodiments of the present invention, when it is determined that there are to-be-optimized regions including an overexposed bright region and/or an underexposed dark region in the target image, for each to-be-optimized region, the X-ray emission dose is adjusted according to the X-ray received dose that meets the imaging quality requirement, the optimized images are acquired based on the X-ray emission dose adjusted to meet the requirement, and the optimized images are then added and synthesized to obtain the X-ray image that meets the imaging quality requirement as a whole.

In addition, whether there is a to-be-optimized region in the target image is automatically detected by the system, which can improve the intelligence and flexibility of the system application.

The foregoing descriptions are merely exemplary embodiments of the present invention, but are not intended to limit the present invention.

## Claims

1. An X-ray image acquisition method, comprising:
acquiring a captured target image of a target region (S21);
when it is determined that there is at least one to-be-optimized region in the target image, calculating, for each to-be-optimized region, a pixel average of the to-be-optimized region in the target image; determining a to-be-optimized region received dose corresponding to the pixel average of the to-be-optimized region according to a relationship between a predetermined pixel average and an X-ray received dose of an X-ray receiver; adjusting an X-ray emission dose of an X-ray source according to a principle of making the to-be-optimized region received dose reach a predetermined X-ray reference received dose, and acquiring an optimized image of the target region captured based on the X-ray emission dose adjusted to meet a requirement (S22~S24), wherein the at least one to-be-optimized region comprises an overexposed bright region and/or an underexposed dark region; and
adding and synthesizing at least one optimized image corresponding to the at least one to-be-optimized region, or adding and synthesizing at least one optimized image corresponding to the at least one to-be-optimized region and the target image, to obtain an X-ray image of the target region (S25).

2. The X-ray image acquisition method according to claim 1, wherein the at least one to-be-optimized region comprises an overexposed bright region and an underexposed dark region; and the calculating, for each to-be-optimized region, a pixel average of the to-be-optimized region in the target image comprises:
calculating a histogram of the target image;
finding a global threshold of the histogram by using a threshold segmentation method; and
calculating, based on the global threshold, an average of pixels in the histogram with values less than the global threshold, to obtain a dark region pixel average; and calculating an average of pixels in the histogram with values greater than the global threshold, to obtain a bright region pixel average.

3. The X-ray image acquisition method according to claim 2, wherein the adding and synthesizing at least one optimized image corresponding to the at least one to-be-optimized region, or adding and synthesizing at least one optimized image corresponding to the at least one to-be-optimized region and the target image comprises:
entirely superimposing the at least one optimized image corresponding to the at least one to-be-optimized region, or synthesizing image parts corresponding to respective to-be-optimized regions in optimized images corresponding to different to-be-optimized regions.

4. The X-ray image acquisition method according to claim 1, wherein the at least one to-be-optimized region comprises an overexposed bright region and/or an underexposed dark region; and the calculating, for each to-be-optimized region, a pixel average of the to-be-optimized region in the target image comprises:
calculating an average of pixels in the target image with brightness values greater than a first brightness threshold, to obtain a bright region pixel average; and/or
calculating an average of pixels in the target image with brightness values less than a second brightness threshold, to obtain a dark region pixel average, wherein the second brightness threshold is less than or equal to the first brightness threshold.

5. The X-ray image acquisition method according to claim 4, wherein the at least one to-be-optimized region comprises an overexposed bright region and an underexposed dark region, and the second brightness threshold is equal to the first brightness threshold; and the adding and synthesizing at least one optimized image corresponding to the at least one to-be-optimized region, or adding and synthesizing at least one optimized image corresponding to the at least one to-be-optimized region and the target image comprises: entirely superimposing the at least one optimized image corresponding to the at least one to-be-optimized region, or synthesizing image parts corresponding to respective to-be-optimized regions in optimized images corresponding to different to-be-optimized regions; or
the at least one to-be-optimized region comprises an overexposed bright region and an underexposed dark region, and the second brightness threshold is less than the first brightness threshold; or the at least one to-be-optimized region comprises an overexposed bright region or an underexposed dark region, and either the bright region or the dark region is part of the target image; and the adding and synthesizing at least one optimized image corresponding to the at least one to-be-optimized region, or adding and synthesizing at least one optimized image corresponding to the at least one to-be-optimized region and the target image comprises: entirely superimposing the at least one optimized image corresponding to the at least one to-be-optimized region and the target image, or synthesizing image parts corresponding to respective to-be-optimized regions in optimized images corresponding to different to-be-optimized regions and image parts in the target image other than the at least one to-be-optimized region.

6. The X-ray image acquisition method according to any one of claims 1 to 5, further comprising:
detecting the pixels in the target image with the brightness values greater than the preset first brightness threshold to obtain overexposed pixels, and determining that there is an overexposed bright region in the target image when a quantity of the overexposed pixels reaches a preset first quantity threshold; and/or
detecting the pixels in the target image with the brightness values less than the preset second brightness threshold to obtain over-dark pixels, and determining that there is a to-be-optimized underexposed dark region in the target image when a quantity of the over-dark pixels reaches a preset second quantity threshold, wherein the second brightness threshold is less than or equal to the first brightness threshold.

7. An X-ray image acquisition system, comprising:
a first unit (510), configured to acquire a captured target image of a target region;
a second unit (520), configured to, when it is determined that there is at least one to-be-optimized region in the target image, calculate, for each to-be-optimized region, a pixel average of the to-be-optimized region in the target image; determine a to-be-optimized region received dose corresponding to the pixel average of the to-be-optimized region according to a relationship between a predetermined pixel average and an X-ray received dose of an X-ray receiver; and adjust an X-ray emission dose of an X-ray source according to a principle of making the to-be-optimized region received dose reach a predetermined X-ray reference received dose, and acquire an optimized image of the target region captured based on the X-ray emission dose adjusted to meet a requirement, wherein the at least one to-be-optimized region comprises an overexposed bright region and/or an underexposed dark region; and
a third unit (530), configured to add and synthesize at least one optimized image corresponding to the at least one to-be-optimized region, or add and synthesize at least one optimized image corresponding to the at least one to-be-optimized region and the target image, to obtain an X-ray image of the target region.

8. The X-ray image acquisition system according to claim 7, wherein the at least one to-be-optimized region comprises an overexposed bright region and an underexposed dark region; and the second unit (520) is configured to calculate a histogram of the target image; find a global threshold of the histogram by using a threshold segmentation method; and calculate, based on the global threshold, an average of pixels in the histogram with values less than the global threshold, to obtain a dark region pixel average; and calculate an average of pixels in the histogram with values greater than the global threshold, to obtain a bright region pixel average.

9. The X-ray image acquisition system according to claim 8, wherein the third unit (530) is configured to entirely superimpose the at least one optimized image corresponding to the at least one to-be-optimized region, or synthesize image parts corresponding to respective to-be-optimized regions in optimized images corresponding to different to-be-optimized regions, to obtain the X-ray image of the target region.

10. The X-ray image acquisition system according to claim 7, wherein the at least one to-be-optimized region comprises an overexposed bright region and/or an underexposed dark region; and the second unit (520) is configured to calculate an average of pixels in the target image with brightness values greater than a first brightness threshold, to obtain a bright region pixel average; and/or calculate an average of pixels in the target image with brightness values less than a second brightness threshold, to obtain a dark region pixel average.

11. The X-ray image acquisition system according to claim 10, wherein the at least one to-be-optimized region comprises an overexposed bright region and an underexposed dark region, and the second brightness threshold is equal to the first brightness threshold; and the third unit (530) is configured to entirely superimpose the at least one optimized image corresponding to the at least one to-be-optimized region, or synthesize image parts corresponding to respective to-be-optimized regions in optimized images corresponding to different to-be-optimized regions, to obtain the X-ray image of the target region; or
the at least one to-be-optimized region comprises an overexposed bright region and an underexposed dark region, and the second brightness threshold is less than the first brightness threshold; or the at least one to-be-optimized region comprises an overexposed bright region or an underexposed dark region, and either the bright region or the dark region is part of the target image; and the third unit (530) is configured to entirely superimpose the at least one optimized image corresponding to the at least one to-be-optimized region and the target image, or synthesize image parts corresponding to respective to-be-optimized regionsin optimized images corresponding to different to-be-optimized regions and image parts in the target image other than the at least one to-be-optimized region.

12. The X-ray image acquisition system according to any one of claims 7 to 11, further comprising: a fourth unit (540), configured to detect the pixels in the target image with the brightness values greater than the preset first brightness threshold to obtain overexposed pixels, and determine that there is an overexposed bright region in the target image when a quantity of the overexposed pixels reaches a preset first quantity threshold; and/or detect the pixels in the target image with the brightness values less than the preset second brightness threshold to obtain over-dark pixels, and determine that there is an underexposed dark region in the target image when a quantity of the over-dark pixels reaches a preset second quantity threshold, wherein the second brightness threshold is less than or equal to the first brightness threshold.

13. An X-ray image acquisition system, comprising: at least one memory (61) and at least one processor (62), wherein
the at least one memory (61) is configured to store a computer program; and
the at least one processor (62) is configured to call the computer program stored in the at least one memory (61) to perform the X-ray image acquisition method according to any one of claims 1 to 6.

14. An X-ray machine, comprising the X-ray image acquisition system according to any one of claims 7 to 13.

15. A computer-readable storage medium, storing a computer program, wherein the computer program is executable by a processor to implement the X-ray image acquisition method according to any one of claims 1 to 6.

## Patentansprüche

1. Röntgenbilderfassungsverfahren, umfassend:
Erfassen eines aufgenommenen Zielbildes einer Zielregion (S21);
wenn bestimmt wird, dass zumindest eine zu optimierende Region in dem Zielbild vorhanden ist, Berechnen eines Pixelmittelwerts der zu optimierenden Region in dem Zielbild für jede zu optimierende Region; Bestimmen einer empfangenen Dosis der zu optimierenden Region entsprechend dem Pixelmittelwert der zu optimierenden Region gemäß einer Beziehung zwischen dem vorbestimmten Pixelmittelwert und einer empfangenen Röntgendosis eines Röntgenempfängers; Anpassen einer Röntgenemissionsdosis einer Röntgenquelle gemäß einem Prinzip, das bewirkt, dass die empfangene Dosis der zu optimierenden Region eine vorbestimmte empfangene Röntgenreferenzdosis erreicht, und Erfassen eines optimierten Bildes der Zielregion, das basierend auf der Röntgenemissionsdosis aufgenommen wurde, die angepasst wurde, um eine Anforderung zu erfüllen (S22-S24), wobei die zumindest eine zu optimierende Region eine überbelichtete helle Region und/oder eine unterbelichtete dunkle Region umfasst; und
Addieren und Synthetisieren von zumindest einem optimierten Bild entsprechend der zumindest einen zu optimierenden Region, oder Addieren und Synthetisieren von zumindest einem optimierten Bild entsprechend der zumindest einen zu optimierenden Region und dem Zielbild, um ein Röntgenbild der Zielregion zu erhalten (S25).

2. Röntgenbilderfassungsverfahren nach Anspruch 1, wobei die zumindest eine zu optimierende Region eine überbelichtete helle Region und eine unterbelichtete dunkle Region umfasst; und das Berechnen eines Pixelmittelwerts der zu optimierenden Region in dem Zielbild für jede zu optimierende Region umfasst:
Berechnen eines Histogramms des Zielbildes;
Finden eines globalen Schwellenwerts des Histogramms unter Verwendung eines Schwellenwertsegmentierungsverfahrens; und
Berechnen eines Mittelwerts der Pixel in dem Histogramm basierend auf dem globalen Schwellenwert mit Werten unter dem globalen Schwellenwert, um einen Pixelmittelwert der dunklen Region zu erhalten; und Berechnen eines Mittelwerts der Pixel in dem Histogramm mit Werten über dem globalen Schwellenwert, um einen Pixelmittelwert der hellen Region zu erhalten.

3. Röntgenbilderfassungsverfahren nach Anspruch 2, wobei das Addieren und Synthetisieren von zumindest einem optimierten Bild entsprechend der zumindest einen zu optimierenden Region, oder Addieren und Synthetisieren von zumindest einem optimierten Bild entsprechend der zumindest einen zu optimierenden Region und dem Zielbild umfasst:
gänzliches Überlagern des zumindest einen optimierten Bildes entsprechend der zumindest einen zu optimierenden Region, oder Synthetisieren von Bildteilen entsprechend den jeweiligen zu optimierenden Regionen in optimierten Bildern entsprechend unterschiedlichen zu optimierenden Regionen.

4. Röntgenbilderfassungsverfahren nach Anspruch 1, wobei die zumindest eine zu optimierende Region eine überbelichtete helle Region und/oder eine unterbelichtete dunkle Region umfasst; und das Berechnen eines Pixelmittelwerts der zu optimierenden Region in dem Zielbild für jede zu optimierende Region umfasst:
Berechnen eines Mittelwerts der Pixel in dem Zielbild mit Helligkeitswerten über einem ersten Helligkeitsschwellenwert, um einen Pixelmittelwert der hellen Region zu erhalten; und/oder
Berechnen eines Mittelwerts der Pixel in dem Zielbild mit Helligkeitswerten unter einem zweiten Helligkeitsschwellenwert, um einen Pixelmittelwert der dunklen Region zu erhalten, wobei der zweite Helligkeitsschwellenwert kleiner als oder gleich dem ersten Helligkeitsschwellenwert ist.

5. Röntgenbilderfassungsverfahren nach Anspruch 4, wobei die zumindest eine zu optimierende Region eine überbelichtete helle Region und eine unterbelichtete dunkle Region umfasst, und der zweite Helligkeitsschwellenwert gleich dem ersten Helligkeitsschwellenwert ist; und das Addieren und Synthetisieren von zumindest einem optimierten Bild entsprechend der zumindest einen zu optimierten Region oder Addieren und Synthetisieren von zumindest einem optimierten Bild entsprechend der zumindest einen zu optimierenden Region und dem Zielbild umfasst: gänzliches Überlagern des zumindest einen optimierten Bildes entsprechend der zumindest einen zu optimierenden Region, oder Synthetisieren von Bildteilen entsprechend jeweiligen zu optimierenden Regionen in optimierten Bildern entsprechend unterschiedlichen zu optimierenden Regionen; oder
die zumindest eine zu optimierende Region eine überbelichtete helle Region und eine unterbelichtete dunkle Region umfasst, und der zweite Helligkeitsschwellenwert kleiner als der erste Helligkeitsschwellenwert ist; oder die zumindest eine zu optimierende Region eine überbelichtete helle Region oder eine unterbelichtete dunkle Region umfasst, und entweder die helle Region oder die dunkle Region Teil des Zielbildes ist; und das Addieren und Synthetisieren von zumindest einem optimierten Bild entsprechend der zumindest einen zu optimierenden Region oder Addieren und Synthetisieren von zumindest einem optimierten Bild entsprechend der zumindest einen zu optimierenden Region und dem Zielbild umfasst: gänzliches Überlagern des zumindest einen optimierten Bildes entsprechend der zumindest einen zu optimierenden Region und dem Zielbild, oder Synthetisieren von Bildteilen entsprechend jeweiligen zu optimierenden Regionen in optimierten Bildern entsprechend unterschiedlichen zu optimierenden Regionen und Bildteilen in dem Zielbild, die von der zumindest einen zu optimierenden Region verschieden sind.

6. Röntgenbilderfassungsverfahren nach einem der Ansprüche 1 bis 5, des Weiteren umfassend:
Detektieren der Pixel in dem Zielbild mit den Helligkeitswerten über dem vorgegebenen ersten Helligkeitsschwellenwert, um überbelichtete Pixel zu erhalten, und Bestimmen, dass eine überbelichtete helle Region in dem Zielbild vorliegt, wenn eine Menge der überbelichteten Pixel einen vorgegebenen ersten Mengenschwellenwert erreicht; und/oder
Detektieren der Pixel in dem Zielbild mit den Helligkeitswerten unter dem vorgegebenen zweiten Helligkeitsschwellenwert, um übermäßig dunkle Pixel zu erhalten, und Bestimmen, dass eine zu optimierende unterbelichtete dunkle Region in dem Zielbild vorhanden ist, wenn eine Menge der übermäßig dunklen Pixel einen vorgegebenen zweiten Mengenschwellenwert erreicht, wobei der zweite Helligkeitsschwellenwert kleiner als oder gleich dem ersten Helligkeitsschwellenwert ist.

7. Röntgenbilderfassungssystem, umfassend:
eine erste Einheit (510), die ausgelegt ist, um ein aufgenommenes Zielbild einer Zielregion zu erfassen;
eine zweite Einheit (520), die ausgelegt ist, um, wenn bestimmt wird, dass zumindest eine zu optimierende Region in dem Zielbild vorhanden ist, einen Pixelmittelwert der zu optimierenden Region in dem Zielbild für jede zu optimierende Region zu berechnen; eine empfangene Dosis der zu optimierenden Region entsprechend dem Pixelmittelwert der zu optimierenden Region gemäß einer Beziehung zwischen dem vorbestimmten Pixelmittelwert und einer empfangenen Röntgendosis eines Röntgenempfängers zu bestimmen; und eine Röntgenemissionsdosis einer Röntgenquelle gemäß einem Prinzip, das bewirkt, dass die empfangene Dosis der zu optimierenden Region eine vorbestimmte empfangene Röntgenreferenzdosis erreicht, anzupassen und ein optimiertes Bild der Zielregion zu erfassen, das basierend auf der Röntgenemissionsdosis aufgenommen wurde, die angepasst wurde, um eine Anforderung zu erfüllen, wobei die zumindest eine zu optimierende Region eine überbelichtete helle Region und/oder eine unterbelichtete dunkle Region umfasst; und
eine dritte Einheit (530), die ausgelegt ist, um zumindest ein optimiertes Bild entsprechend der zumindest einen zu optimierenden Region zu addieren und zu synthetisieren, oder zumindest ein optimiertes Bild entsprechend der zumindest einen zu optimierenden Region und dem Zielbild zu addieren und zu synthetisieren, um ein Röntgenbild der Zielregion zu erhalten.

8. Röntgenbilderfassungssystem nach Anspruch 7, wobei die zumindest eine zu optimierende Region eine überbelichtete helle Region und eine unterbelichtete dunkle Region umfasst; und die zweite Einheit (520) ausgelegt ist, um ein Histogramm des Zielbildes zu berechnen; einen globalen Schwellenwert des Histogramms unter Verwendung eines Schwellenwertsegmentierungsverfahrens zu finden; und basierend auf dem globalen Schwellenwert einen Mittelwert der Pixel in dem Histogramm mit Werten unter dem globalen Schwellenwert zu berechnen, um einen Pixelmittelwert der dunklen Region zu erhalten; und einen Mittelwert der Pixel in dem Histogramm mit Werten über dem globalen Schwellenwert zu berechnen, um einen Pixelmittelwert der hellen Region zu erhalten.

9. Röntgenbilderfassungssystem nach Anspruch 8, wobei die dritte Einheit (530) ausgelegt ist, um das zumindest eine optimierte Bild entsprechend der zumindest einen zu optimierenden Region gänzlich zu überlagern oder Bildteile entsprechend jeweiligen zu optimierenden Regionen in optimierten Bildern entsprechend unterschiedlichen zu optimierenden Regionen zu synthetisieren, um das Röntgenbild der Zielregion zu erhalten.

10. Röntgenbilderfassungssystem nach Anspruch 7, wobei die zumindest eine zu optimierende Region eine überbelichtete helle Region und/oder eine unterbelichtete dunkle Region umfasst; und die zweite Einheit (520) ausgelegt ist, um einen Mittelwert der Pixel in dem Zielbild mit Helligkeitswerten über einem ersten Helligkeitsschwellenwert zu berechnen, um einen Pixelmittelwert der hellen Region zu erhalten; und/oder einen Mittelwert der Pixel in dem Zielbild mit Helligkeitswerten unter einem zweiten Helligkeitsschwellenwert zu berechnen, um einen Pixelmittelwert der dunklen Region zu erhalten.

11. Röntgenbilderfassungssystem nach Anspruch 10, wobei die zumindest eine zu optimierende Region eine überbelichtete helle Region und eine unterbelichtete dunkle Region umfasst, und der zweite Helligkeitsschwellenwert gleich dem ersten Helligkeitsschwellenwert ist; und die dritte Einheit (530) ausgelegt ist, um das zumindest eine optimierte Bild entsprechend der zumindest einen zu optimierenden Region gänzlich zu überlagern oder Bildteile entsprechend jeweiligen zu optimierenden Regionen in optimierten Bildern entsprechend unterschiedlichen zu optimierenden Regionen zu synthetisieren, um das Röntgenbild der Zielregion zu erhalten; oder
die zumindest eine zu optimierende Region eine überbelichtete helle Region und eine unterbelichtete dunkle Region umfasst, und der zweite Helligkeitsschwellenwert kleiner als der erste Helligkeitsschwellenwert ist; oder die zumindest eine zu optimierende Region eine überbelichtete helle Region oder eine unterbelichtete dunkle Region umfasst, und entweder die helle Region oder die dunkle Region Teil des Zielbildes ist; und die dritte Einheit (530) ausgelegt ist, um das zumindest eine optimierte Bild entsprechend der zumindest einen zu optimierenden Region und dem Zielbild gänzlich zu überlagern, oder Bildteile entsprechend jeweiligen zu optimierenden Regionen in optimierten Bildern entsprechend unterschiedlichen zu optimierenden Regionen und Bildteilen in dem Zielbild, die von der zumindest einen zu optimierten Region verschieden sind, zu synthetisieren.

12. Röntgenbilderfassungssystem nach einem der Ansprüche 7 bis 11, des Weiteren umfassend: eine vierte Einheit (540), die ausgelegt ist, um die Pixel in dem Zielbild mit den Helligkeitswerten über dem vorgegebenen ersten Helligkeitsschwellenwert zu detektieren, um überbelichtete Pixel zu erhalten, und zu bestimmen, dass eine überbelichtete helle Region in dem Zielbild vorhanden ist, wenn eine Menge der überbelichteten Pixel einen vorgegebenen ersten Mengenschwellenwert erreicht; und/oder die Pixel in dem Zielbild mit den Helligkeitswerten unter dem vorgegebenen zweiten Helligkeitsschwellenwert zu detektieren, um übermäßig dunkle Pixel zu erhalten, und zu bestimmen, dass eine unterbelichtete dunkle Region in dem Zielbild vorhanden ist, wenn eine Menge der übermäßig dunklen Pixel einen vorgegebenen zweiten Mengenschwellenwert erreicht, wobei der zweite Helligkeitsschwellenwert kleiner als oder gleich dem ersten Helligkeitsschwellenwert ist.

13. Röntgenbilderfassungssystem, umfassend: zumindest einen Speicher (61) und zumindest einen Prozessor (62), wobei
der zumindest eine Speicher (61) ausgelegt ist, ein Computerprogramm zu speichern; und
der zumindest eine Prozessor (62) ausgelegt ist, um das in dem zumindest einen Speicher (61) gespeicherte Programm aufzurufen, um das Röntgenbilderfassungsverfahren gemäß einem der Ansprüche 1 bis 6 durchzuführen.

14. Röntgengerät, umfassend das Röntgenbilderfassungssystem gemäß einem der Ansprüche 7 bis 13.

15. Computerlesbares Speichermedium, das ein Computerprogramm speichert, wobei das Computerprogramm von einem Prozessor ausgeführt wird, um das Röntgenbilderfassungsverfahren nach einem der Ansprüche 1 bis 6 zu implementieren.

## Revendications

1. Procédé d'acquisition d'images aux rayons X, comprenant :
l'acquisition d'une image cible capturée d'une région cible (S21) ;
quand il est déterminé qu'il y a au moins une région à optimiser dans l'image cible, le calcul, pour chaque région à optimiser, d'une moyenne de pixels de la région à optimiser dans l'image cible ; la détermination d'une dose reçue de région à optimiser correspondant à la moyenne de pixels de la région à optimiser en fonction d'une relation entre une moyenne de pixels prédéterminée et une dose de rayons X reçue d'un récepteur de rayons X ; le réglage d'une dose d'émission de rayons X d'une source de rayons X selon un principe consistant à faire atteindre à la dose reçue de région à optimiser une dose de rayons X reçue de référence prédéterminée, et l'acquisition d'une image optimisée de la région cible capturée sur la base de la dose d'émission de rayons X réglée pour respecter une exigence (S22-S24), l'au moins une région à optimiser comprenant une région claire surexposée et/ou une région sombre sous-exposée ; et
l'ajout et la synthèse d'au moins une image optimisée correspondant à l'au moins une région à optimiser, ou l'ajout et la synthèse d'au moins une image optimisée correspondant à l'au moins une région à optimiser et l'image cible, pour obtenir une image aux rayons X de la région cible (S25).

2. Procédé d'acquisition d'images aux rayons X selon la revendication 1, dans lequel l'au moins une région à optimiser comprend une région claire surexposée et une région sombre sous-exposée ; et le calcul, pour chaque région à optimiser, d'une moyenne de pixels de la région à optimiser dans l'image cible comprend :
le calcul d'un histogramme de l'image cible ;
la recherche d'un seuil global de l'histogramme au moyen d'un procédé de segmentation de seuil ; et
le calcul, sur la base du seuil global, d'une moyenne de pixels dans l'histogramme avec des valeurs inférieures au seuil global, pour obtenir une moyenne de pixels de région sombre ; et le calcul d'une moyenne de pixels dans l'histogramme avec des valeurs supérieures au seuil global, pour obtenir une moyenne de pixels de région claire.

3. Procédé d'acquisition d'images aux rayons X selon la revendication 2, dans lequel l'ajout et la synthèse d'au moins une image optimisée correspondant à l'au moins une région à optimiser, ou l'ajout et la synthèse d'au moins une image optimisée correspondant à l'au moins une région à optimiser et l'image cible comprennent :
la superposition totale de l'au moins une image optimisée correspondant à l'au moins une région à optimiser, ou la synthèse de parties d'image correspondant à des régions à optimiser respectives dans des images optimisées correspondant à différentes régions à optimiser.

4. Procédé d'acquisition d'images aux rayons X selon la revendication 1, dans lequel l'au moins une région à optimiser comprend une région claire surexposée et/ou une région sombre sous-exposée ; et le calcul, pour chaque région à optimiser, d'une moyenne de pixels de la région à optimiser dans l'image cible comprend :
le calcul d'une moyenne de pixels dans l'image cible avec des valeurs de luminosité supérieures à un premier seuil de luminosité, pour obtenir une moyenne de pixels de région claire ; et/ou
le calcul d'une moyenne de pixels dans l'image cible avec des valeurs de luminosité inférieures à un deuxième seuil de luminosité, pour obtenir une moyenne de pixels de région sombre, le deuxième seuil de luminosité étant inférieur ou égal au premier seuil de luminosité.

5. Procédé d'acquisition d'images aux rayons X selon la revendication 4, dans lequel l'au moins une région à optimiser comprend une région claire surexposée et une région sombre sous-exposée, et le deuxième seuil de luminosité est égal au premier seuil de luminosité ; et l'ajout et la synthèse d'au moins une image optimisée correspondant à l'au moins une région à optimiser, ou l'ajout et la synthèse d'au moins une image optimisée correspondant à l'au moins une région à optimiser et l'image cible comprennent : la superposition totale de l'au moins une image optimisée correspondant à l'au moins une région à optimiser, ou la synthèse de parties d'image correspondant à des régions à optimiser respectives dans des images optimisées correspondant à différentes régions à optimiser ; ou
l'au moins une région à optimiser comprend une région claire surexposée et une région sombre sous-exposée, et le deuxième seuil de luminosité est inférieur au premier seuil de luminosité ; ou l'au moins une région à optimiser comprend une région claire surexposée ou une région sombre sous-exposée, et soit la région claire, soit la région sombre fait partie de l'image cible ; et l'ajout et la synthèse d'au moins une image optimisée correspondant à l'au moins une région à optimiser, ou l'ajout et la synthèse d'au moins une image optimisée correspondant à l'au moins une région à optimiser et l'image cible comprennent : la superposition totale de l'au moins une image optimisée correspondant à l'au moins une région à optimiser et l'image cible, ou la synthèse de parties d'image correspondant à des régions à optimiser respectives dans des images optimisées correspondant à différentes régions à optimiser et de parties d'image dans l'image cible autres que l'au moins une région à optimiser.

6. Procédé d'acquisition d'images aux rayons X selon l'une quelconque des revendications 1 à 5, comprenant en outre :
la détection des pixels dans l'image cible avec les valeurs de luminosité supérieures au premier seuil de luminosité prédéfini pour obtenir des pixels surexposés, et la détermination qu'il y a une région claire surexposée dans l'image cible quand une quantité des pixels surexposés atteint un premier seuil de quantité prédéfini ; et/ou
la détection des pixels dans l'image cible avec les valeurs de luminosité inférieures au deuxième seuil de luminosité prédéfini pour obtenir des pixels trop sombres, et la détermination qu'il y a une région sombre sous-exposée à optimiser dans l'image cible quand une quantité des pixels trop sombres atteint un deuxième seuil de quantité prédéfini, le deuxième seuil de luminosité étant inférieur ou égal au premier seuil de luminosité.

7. Système d'acquisition d'images aux rayons X, comprenant :
une première unité (510), configurée pour acquérir une image cible capturée d'une région cible ;
une deuxième unité (520), configurée pour, quand il est déterminé qu'il y a au moins une région à optimiser dans l'image cible, calculer, pour chaque région à optimiser, une moyenne de pixels de la région à optimiser dans l'image cible ; déterminer une dose reçue de région à optimiser correspondant à la moyenne de pixels de la région à optimiser en fonction d'une relation entre une moyenne de pixels prédéterminée et une dose de rayons X reçue d'un récepteur de rayons X ; et régler une dose d'émission de rayons X d'une source de rayons X selon un principe consistant à faire atteindre à la dose reçue de région à optimiser une dose de rayons X reçue de référence prédéterminée, et acquérir une image optimisée de la région cible capturée sur la base de la dose d'émission de rayons X réglée pour respecter une exigence, l'au moins une région à optimiser comprenant une région claire surexposée et/ou une région sombre sous-exposée ; et
une troisième unité (530), configurée pour ajouter et synthétiser au moins une image optimisée correspondant à l'au moins une région à optimiser, ou ajouter et synthétiser au moins une image optimisée correspondant à l'au moins une région à optimiser et l'image cible, pour obtenir une image aux rayons X de la région cible.

8. Système d'acquisition d'images aux rayons X selon la revendication 7, dans lequel l'au moins une région à optimiser comprend une région claire surexposée et une région sombre sous-exposée ; et la deuxième unité (520) est configurée pour calculer un histogramme de l'image cible ; rechercher un seuil global de l'histogramme en utilisant un procédé de segmentation de seuil ; et calculer, sur la base du seuil global, une moyenne de pixels dans l'histogramme avec des valeurs inférieures au seuil global, pour obtenir une moyenne de pixels de région sombre ; et calculer une moyenne de pixels dans l'histogramme avec des valeurs supérieures au seuil global, pour obtenir une moyenne de pixels de région claire.

9. Système d'acquisition d'images aux rayons X selon la revendication 8, dans lequel la troisième unité (530) est configurée pour superposer totalement l'au moins une image optimisée correspondant à l'au moins une région à optimiser, ou synthétiser des parties d'image correspondant à des régions à optimiser respectives dans des images optimisées correspondant à différentes régions à optimiser, pour obtenir l'image aux rayons X de la région cible.

10. Système d'acquisition d'images aux rayons X selon la revendication 7, dans lequel l'au moins une région à optimiser comprend une région claire surexposée et/ou une région sombre sous-exposée ; et la deuxième unité (520) est configurée pour calculer une moyenne de pixels dans l'image cible avec des valeurs de luminosité supérieures à un premier seuil de luminosité, pour obtenir une moyenne de pixels de région claire ; et/ou calculer une moyenne de pixels dans l'image cible avec des valeurs de luminosité inférieures à un deuxième seuil de luminosité, pour obtenir une moyenne de pixels de région sombre.

11. Système d'acquisition d'images aux rayons X selon la revendication 10, dans lequel l'au moins une région à optimiser comprend une région claire surexposée et une région sombre sous-exposée, et le deuxième seuil de luminosité est égal au premier seuil de luminosité ; et la troisième unité (530) est configurée pour superposer totalement l'au moins une image optimisée correspondant à l'au moins une région à optimiser, ou synthétiser des parties d'image correspondant à des régions à optimiser respectives dans des images optimisées correspondant à différentes régions à optimiser, pour obtenir l'image aux rayons X de la région cible ; ou
l'au moins une région à optimiser comprend une région claire surexposée et une région sombre sous-exposée, et le deuxième seuil de luminosité est inférieur au premier seuil de luminosité ; ou l'au moins une région à optimiser comprend une région claire surexposée ou une région sombre sous-exposée, et soit la région claire, soit la région sombre fait partie de l'image cible ; et la troisième unité (530) est configurée pour superposer totalement l'au moins une image optimisée correspondant à l'au moins une région à optimiser et l'image cible, ou synthétiser des parties d'image correspondant à des régions à optimiser respectives dans des images optimisées correspondant à différentes régions à optimiser et des parties d'image dans l'image cible autres que l'au moins une région à optimiser.

12. Système d'acquisition d'images aux rayons X selon l'une quelconque des revendications 7 à 11, comprenant en outre : une quatrième unité (540), configurée pour détecter les pixels dans l'image cible avec les valeurs de luminosité supérieures au premier seuil de luminosité prédéfini pour obtenir des pixels surexposés, et déterminer qu'il y a une région claire surexposée dans l'image cible quand une quantité des pixels surexposés atteint un premier seuil de quantité prédéfini ; et/ou détecter les pixels dans l'image cible avec les valeurs de luminosité inférieures au deuxième seuil de luminosité prédéfini pour obtenir des pixels trop sombres, et déterminer qu'il y a une région sombre sous-exposée dans l'image cible quand une quantité des pixels trop sombres atteint un deuxième seuil de quantité prédéfini, le deuxième seuil de luminosité étant inférieur ou égal au premier seuil de luminosité.

13. Système d'acquisition d'images aux rayons X, comprenant : au moins une mémoire (61) et au moins un processeur (62), dans lequel
l'au moins une mémoire (61) est configurée pour stocker un programme informatique ; et
l'au moins un processeur (62) est configuré pour appeler le programme informatique stocké dans l'au moins une mémoire (61) pour effectuer le procédé d'acquisition d'images aux rayons X selon l'une quelconque des revendications 1 à 6.

14. Machine à rayons X, comprenant le système d'acquisition d'images aux rayons X selon l'une quelconque des revendications 7 à 13.

15. Support de stockage lisible par ordinateur stockant un programme informatique, le programme informatique étant exécutable par un processeur pour mettre en oeuvre le procédé d'acquisition d'images aux rayons X selon l'une quelconque des revendications 1 à 6.
